# EUROPÄISCHE PATENTSCHRIFT

(11) **EP 3 166 668 B1**
(45) Veröffentlichungstag und Bekanntmachung des Hinweises auf die Patenterteilung: **04.09.2019**
(21) Anmeldenummer: 15745412.5
(22) Anmeldetag: 10.07.2015
(51) Int. Cl.: A61M 15/06, A24F 47/00, A61K 45/06, A61K 41/00, A61M 31/00, A61J 7/00, A61J 1/06

(54) **VORRICHTUNG ZUM APPLIZIEREN VON FLÜSSIGKEITEN**
DEVICE FOR ADMINISTERING LIQUIDS
DISPOSITIF D'APPLICATION DE LIQUIDES

(30) Priorität: 11.07.2014 DE 202014103195 U
(43) Veröffentlichungstag der Anmeldung: 17.05.2017
(73) Patentinhaber: Gross, Marianna, 66557 IIlingen (DE)
(72) Erfinder: Gross, Marianna, 66557 IIlingen (DE)
(74) Vertreter: Hoffmann Eitle
(86) Internationale Anmeldenummer: PCT/EP2015/065846
(87) Internationale Veröffentlichungsnummer: WO 2016/005562

(56) Entgegenhaltungen:
- DE-U1-202007 017 543
- DE-U1-202008 001 917
- DE-U1-202013 103 370
- US-A1- 2006 108 374

## Beschreibung

### Technisches Gebiet

Die Erfindung betrifft eine Vorrichtung zum Applizieren von Flüssigkeiten, insbesondere zum Applizieren von homöopathischen Flüssigkeiten zur Raucherentwöhnung. Ferner betrifft die Erfindung die Verwendung der Vorrichtung zum Applizieren von Flüssigkeiten für ein Arzneimittel zur Raucherentwöhnungstherapie.

### Hintergrund

Für viele Raucher stellt sich im Rahmen der Raucherentwöhnung die Problematik, dass sie die Gewohnheit haben, eine Zigarette in der Hand zu halten bzw. in den Mund zu nehmen. Selbst wenn sie nicht mehr rauchen, haben sie aufgrund einer psychischen Abhängigkeit noch lange das Bedürfnis, einen Gegenstand in die Hand zu nehmen oder auch in den Mund zu nehmen.

Eine Lösung hierfür stellen sogenannte elektrische Zigaretten dar. In ihnen wird eine Lösung verdampft, die dann inhaliert werden kann. Allerdings ist teilweise die verdampfte Lösung auch nikotinhaltig, so dass die Nikotinabhängigkeit hiermit nicht bekämpft werden kann. Zudem werden elektrische Zigaretten in manchen Ländern zunehmend kritisch betrachtet und sie unterliegen ähnlichen Beschränkungen in öffentlichen Bereichen wie normale Zigaretten.

### Darstellung der Erfindung

Der Erfindung liegt die Aufgabe zugrunde, eine Vorrichtung zum Applizieren von Flüssigkeiten bereitzustellen, die auf einfache Weise und in reproduzierbaren Mengen eine orale Applikation geringer Mengen von Flüssigkeiten ermöglicht. Ferner liegt der Erfindung liegt die Aufgabe zugrunde, eine Vorrichtung zum Applizieren von Flüssigkeiten zur Raucherentwöhnung bereitzustellen, die auf einfache Weise und in reproduzierbaren Mengen eine orale Applikation geringer Mengen von Flüssigkeiten zur Raucherentwöhnung ermöglicht und die als Ersatz für elektrische Zigaretten eingesetzt werden kann.

Diese Aufgaben werden erfindungsgemäß durch die Vorrichtung nach Anspruch 1 und durch die Vorrichtung nach Anspruch 2 gelöst.

Die erfindungsgemäße Vorrichtung weist eine zusammendrückbare im Wesentlichen hohlzylindrische Hülle auf, welche an ihrem unteren Ende geschlossen und an ihrem oberen Ende offen ist, wobei in der hohlzylindrischen Hülle ein saugfähiges Element angeordnet ist und ein geschlitzter Stopfen vorgesehen ist, der das offene Ende der hohlzylindrischen Hülle verschließt, und wobei in dem saugfähigen Element ein perforiertes Steigrohr angeordnet ist, das in den Schlitz des Stopfens mündet.

In einer weiteren Ausführungsform weist die erfindungsgemäße Vorrichtung eine feste im Wesentlichen hohlzylindrische Hülle auf, welche an ihrem unteren Ende geschlossen und an ihrem oberen Ende offen ist, wobei in der hohlzylindrischen Hülle ein saugfähiges Element angeordnet ist und ein geschlitzter Stopfen vorgesehen ist, der das offene Ende der hohlzylindrischen Hülle verschließt, und wobei in dem saugfähigen Element ein perforiertes Steigrohr angeordnet ist, das in den Schlitz des Stopfens mündet.

Die erfindungsgemäße Vorrichtung hat somit eine im Wesentlichen zylindrische Form, die im Wesentlichen der äußeren Form einer Zigarette entspricht. Ihr äußeres Erscheinungsbild kann dasjenige einer Zigarette, eines Lippenstiftes oder eines Kugelschreibers oder eines beliebigen anderen länglichen Gegenstandes sein.

Die erfindungsgemäße Vorrichtung kann zur oralen oder zur dermalen Applikation der darin enthaltenen Flüssigkeit vorgesehen sein. Bevorzugt ist die erfindungsgemäße Vorrichtung zur oralen Applikation vorgesehen. Ferner bevorzugt ist die erfindungsgemäße Vorrichtung zur oralen Applikation vorgesehen und liegt in Form einer Zigarette vor. Die dermale Applikation umfasst die Applikation über die Haut und über die Lippen. Für eine dermale Applikation über die Lippen kann die erfindungsgemäße Vorrichtung in Form eines Lippenstifts vorliegen.

Die erfindungsgemäße Vorrichtung kann 1-30 ml und bevorzugt 2-10 ml einer mit der Vorrichtung zu applizierenden Flüssigkeit enthalten.

Die erfindungsgemäße Vorrichtung kann zur Einfach- oder Mehrfachapplikation vorgesehen sein.

Bevorzugt ist die erfindungsgemäße Vorrichtung zur Mehrfachapplikation vorgesehen.

Im Fall der Mehrfachapplikation enthält die erfindungsgemäße Vorrichtung bevorzugt 10 ml der zu applizierenden Flüssigkeit und es wird pro Applikation 1 ml der jeweiligen Flüssigkeit abgegeben. 1 ml entspricht 30 Tropfen, so dass eine Tagesdosis von 5x30 Tropfen einer Menge von ca. 5 ml Flüssigkeit entspricht, welche für eine Tagesdosis homöopathischer Arzneimittel üblich ist.

Sofern die erfindungsgemäße Vorrichtung zur Einfachapplikation vorgesehen ist, kann es sich um eine Einwegvorrichtung handeln. Die mit der Vorrichtung als Einmaldosis applizierbare Flüssigkeitsmenge entspricht in diesem Fall der in der Vorrichtung enthaltenen Flüssigkeitsmenge.

In einer weiteren Ausführungsform kann die zur Einfach- oder Mehrfachapplikation vorgesehene erfindungsgemäße Vorrichtung wieder auffüllbar sein.

Bei Bitterstoffen kann sowohl die Menge größer sein als auch die tägliche Maximaldosierung, zum Beispiel ein Füllvolumen von 5-20 ml und ein pro Applikation abgegebenes Volumen von 1-3 ml.

Der Innenbereich der hohlzylindrischen Hülle der erfindungsgemäßen Vorrichtung ist vorzugsweise gänzlich mit saugfähigem Material ausgekleidet, das die zu applizierende Flüssigkeit aufnimmt und speichert.

Im Fall einer zusammendrückbaren Hülle wird die in dem saugfähigen Element der erfindungsgemäßen Vorrichtung enthaltene Flüssigkeit durch Zusammendrücken der hohlzylindrischen Hülle zu dem geschlitzten Stopfen gedrückt, wo sie - beispielsweise unter Tropfenbildung auf dem geschlitzten Stopfen - zur Applikation zur Verfügung steht. Wird die hohlzylindrische Hülle nicht mehr zusammengedrückt, wird die Flüssigkeit wieder von dem saugfähigen Material aufgenommen. Aufgrund der Schlitzung des Stopfens verschließt sich die Öffnung selbsttätig.

Sofern die erfindungsgemäße Vorrichtung eine feste Hülle aufweist, erfolgt die Applikation mittels Saugen an dem geschlitzten Stopfen, der das offene Ende der hohlzylindrischen Hülle verschließt.

Das Steigrohr erleichtert das Aufsteigen der Flüssigkeit zum Stopfen hin. Beim Zusammendrücken der hohlzylindrischen Hülle oder beim Saugen an dem geschlitzten Stopfen tritt die Flüssigkeit durch die Perforationen des Steigrohres in dieses ein und steigt darin bis zum Stopfen auf, wo sie durch den Schlitz austritt.

Eine bevorzugte Ausbildung der Erfindung besteht darin, dass die hohlzylindrische Hülle aus Kunststoff besteht.

Kunststoff hat den Vorteil, flüssigkeitsbeständig zu sein und kann bedruckt werden, um der Vorrichtung beispielsweise ein zigarettenähnliches Aussehen zu verleihen.

Es liegt im Rahmen der Erfindung, dass das saugfähige Element aus Schaumstoff besteht. Schaumstoff ist hochgradig saugfähig und in hygienischer Hinsicht unbedenklich. Alternativ kann auch ein anderes hygienisch unbedenkliches und saugfähiges Material, wie z.B. Watte, verwendet werden.

Weiterhin ist erfindungsgemäß vorgesehen, dass der Stopfen vorzugsweise im Wesentlichen halbkugelförmig ausgebildet ist.

Es ist vorteilhaft, dass der Stopfen aus einem gummielastischen Material besteht.

Bei einem mit einem Schlitz versehenen, im Wesentlichen halbkugelförmigen Stopfen aus gummielastischem Material ergibt sich eine selbstschließende Wirkung.

Weiterhin ist zur Erfindung gehörig, dass das Steigrohr über seine gesamte Länge perforiert ist.

Weiterhin ist es erfindungsgemäß, dass eine Schutzkappe zum Verschließen der Vorrichtung vorgesehen ist.

Die Schutzkappe wird über den Gummistopfen auf die hohlzylindrische Hülle geschoben, um insbesondere eine Verunreinigung des Stopfens zu verhindern und sicherzustellen, dass nicht durch versehentliche Druckausübung Flüssigkeit aus der Vorrichtung austritt.

Der Begriff "im Wesentlichen", so wie er in der Beschreibung und den Ansprüchen im Zusammenhang mit der erfindungsgemäßen Vorrichtung verwendet wird, beschreibt die (herstellungs-)technisch bedingte Abweichung der Hülle der erfindungsgemäßen Vorrichtung von einer idealen hohlzylindrischen bzw. zylindrischen Form sowie die (herstellungs-)technisch bedingte Abweichung des Stopfens der erfindungsgemäßen Vorrichtung von einer idealen halbkugelförmigen Form.

Bei der Flüssigkeit kann es sich um ein flüssiges Arzneimittel, beispielsweise eine nikotinhaltige Lösung zur Nikotinabgabe im Zuge einer Raucherentwöhnung, oder eine homöopathische Lösung handeln, die die Raucherentwöhnung unterstützt. Ebenso ist es möglich, dass die Flüssigkeit eine Flüssigkeit auf Basis eines oder mehrerer ätherischer Öle, von Geschmacks-, Geruchs- oder Bitterstoffen ist. Schließlich kann die Flüssigkeit auch ein beliebiges Getränk, beispielsweise ein Fruchtsaft, sein. Es kann sich bei der Flüssigkeit auch um ein Gel oder einen Sirup handeln.

Flüssigkeiten, bei denen es sich um homöopathische Lösungen zur Raucherentwöhnung handelt und die zur Applikation mit der erfindungsgemäßen Vorrichtung geeignet sind, können 1,5 bis 2 Gew.-% Passiflora incarnata D30, 0,75 bis 1,25 Gew.-% Rhus toxicodendron D60, 0,75 bis 1,25 Gew.-% Ignatia D30, 1 bis 1,5 Gew.-% Cactus D30, 0,25 bis 0,75 Gew.-% Panax Ginseng D30, 0,75 bis 1,25 Gew.-% Pulsatilla D23, 1,5 bis 2 Gew.-% Lobelia inflata D60, 0,5 bis 1 Gew.-% Stramonium und 1,5 bis 2,5 Gew.-% Nux Moschata D30 enthalten. Die Flüssigkeiten können ferner eine isotonische NaCI-Lösung und/oder Wasser enthalten. Zudem können die Flüssigkeiten 1 bis 1,5 Gew.-% Robinia pseudoacacia D30, 1,5 bis 2 Gew.-% Zincum metallicum D26, 0,75 bis 1,25 Gew.-% Sulphur D6, 0,75 bis 1,25 Gew.-% Iodum D30, 0,75 bis 1,25 Gew.-% Chamomilla D30, 1 bis 2 Gew.-% Phosphorus D28, 0,5 bis 1 Gew.-% Nux vomica D30 und/oder 1 bis 1,5 Gew.-% Agnus Cactus D4 enthalten.

Insbesondere können folgende Flüssigkeiten mittels der erfindungsgemäßen Vorrichtung appliziert werden:

**Lösung 1**

| | |
|---|---|
| Passiflora incarnata D30 | 1,5 g |
| Rhus toxicodendron D60 | 0,8 g |
| Ignatia D30 | 1,0 g |
| Zincum metallicum D26 | 1,8 g |
| Cactus D30 | 1,1 g |
| Sulphur D6 | 0,9 g |
| Panax Ginseng D30 | 0,4 g |
| Pulsatilla D23 | 1,1 g |
| Iodum D30 | 0,9 g |
| Lobelia inflata D60 | 1,6 g |
| Stramonium D30 | 1,1 g |
| Nux Moschata D30 | 1,8 g |
| Agnus Cactus D4 | 1,1 g |
| Destilliertes Wasser | 80,0 g |

**Lösung 2**

| | |
|---|---|
| Passiflora incarnata D30 | 1,5 g |
| Rhus toxicodendron D60 | 0,8 g |
| Robinia pseudoacacia D30 | 1,1 g |
| Ignatia D30 | 1,0 g |
| Zincum metallicum D26 | 1,8 g |
| Cactus D30 | 1,1 g |
| Sulphur D6 | 0,9 g |
| Panax Ginseng D30 | 0,4 g |
| Pulsatilla D23 | 1,1 g |
| Lobelia inflata D60 | 1,6 g |
| Chamomilla D30 | 0,9 g |
| Phosphorus D28 | 1,4 g |
| Stramonium D30 | 1,1 g |
| Nux vomica D30 | 0,7 g |
| Nux Moschata D30 | 1,8 g |
| Isotonische NaCI-Lösung | 80,0 g |

Bevorzugt kann ferner die folgende Flüssigkeit mittels der erfindungsgemäßen Vorrichtung appliziert werden:

**Lösung 3**

| | |
|---|---|
| Hypericum perforatum D1 | 0,01 g |
| Valeriana officinalis D1 | 0,01 g |
| Lupulinum (HAB 34) D1 | 0,01 g |
| [HAB, V. 4a, D1 mit Ethanol 86 % (m/m)] | |
| Vitex agnus-castus D1 | 0,01 g |
| Avena sativa D1 | 0,01 g |
| Coffea arabica D1 | 0,01 g |
| Ethanol 62 % (m/m) | ad 10 g (entsprechend 12 ml) |

In einer weiteren Ausführungsform betrifft die vorliegende Erfindung die Verwendung der erfindungsgemäßen Vorrichtung enthaltend eine der zuvor beschriebenen Lösungen 1-3 als Arzneimittel zur Raucherentwöhnungstherapie.

Die vorliegende Erfindung betrifft ferner ein Verfahren zur Herstellung der erfindungsgemäßen Vorrichtung.

### Kurze Beschreibung der Abbildungen

Fig. 1 zeigt eine geschnittene Darstellung durch eine erste erfindungsgemäße Vorrichtung zur Applikation von Flüssigkeiten.
Fig. 2 eine geschnittene Darstellung durch eine zweite erfindungsgemäße Vorrichtung zur Applikation von Flüssigkeiten.

### Wege zur Ausführung der Erfindung

Bevorzugte Ausführungsformen der erfindungsgemäßen Vorrichtung werden im Folgenden unter Bezug auf die beigefügten Zeichnungen und die darin verwendeten Bezugszeichen erläutert.

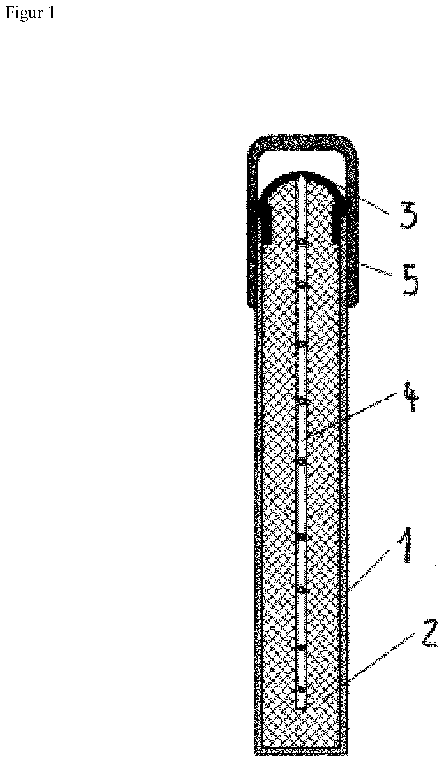

Die in Fig. 1 gezeigte Vorrichtung weist eine zusammendrückbare hohlzylindrische Hülle (1) auf, in der ein saugfähiges Element (2) angeordnet ist. Die hohlzylindrische Hülle (1) ist an ihrem einen Ende geschlossen und an ihrem anderen Ende offen ausgebildet. Sie besteht vorzugsweise aus Kunststoff. Das saugfähige Element (2) kann beispielsweise aus Schaumstoff oder aus Watte bestehen. Es wird mit der zu applizierenden Flüssigkeit getränkt.

Das offene Ende der hohlzylindrischen Hülle (1) ist durch einen geschlitzten Stopfen (3) verschlossen, wobei der Stopfen (3) vorzugsweise im Wesentlichen halbkugelförmig ausgebildet ist. Der Schlitz des Stopfens (3) ist als Längsschlitz ausgebildet, der vorzugsweise durch das Zentrum des Stopfens (3) verläuft. Der Stopfen (3) besteht vorzugsweise aus einem gummielastischen Material, beispielsweise aus natürlichem oder synthetischem Kautschuk.

Im Zentrum des saugfähigen Elementes (2) ist ein perforiertes Steigrohr (4) angeordnet, das axial in der hohlzylindrischen Hülle (1) verläuft und in den Schlitz des Stopfens (3) mündet.

Zum Verschließen der Vorrichtung dient eine Schutzkappe (5), die über den Stopfen (3) gestülpt wird und deren freies Ende die hohlzylindrische Hülle (1) umschließt.

Die Vorrichtung wird von dem Benutzer vor Gebrauch durch Entfernen der Schutzkappe (5) geöffnet. Dann wird Druck auf die Seitenwände der hohlzylindrischen Hülle (1) ausgeübt, wodurch in dem saugfähigen Element (2) gespeicherte Flüssigkeit durch die Perforationen in das Steigrohr (4) eintritt, durch dieses bis zum Stopfen (3) aufsteigt und aus dessen Schlitz austritt, beispielsweise unter Tropfenbildung. Die austretende Flüssigkeit wird oral aufgenommen und die Vorrichtung wieder verschlossen. Sobald kein Druck mehr auf die Seitenwände der hohlzylindrischen Hülle (1) ausgeübt wird, geht die Vorrichtung selbsttätig in die Ausgangsstellung zurück.

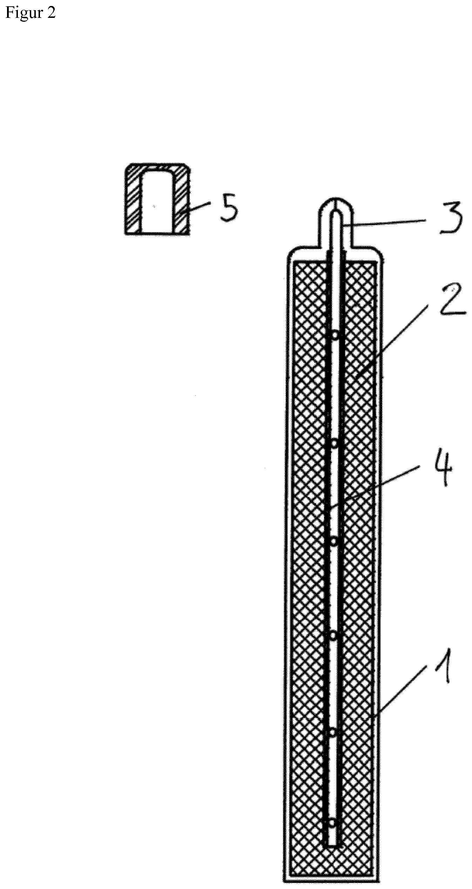

Der Aufbau der Vorrichtung gemäß Fig. 2 entspricht im Wesentlichen demjenigen der in Fig. 1 dargestellten Vorrichtung, unterscheidet sich jedoch hiervon in der Ausbildung des Stopfens (3). Während dieser sich bei der in Fig. 1 gezeigten Vorrichtung im Wesentlichen halbkugelförmig direkt an die Hülle (1) anschließt, weist er bei der Vorrichtung gemäß Fig. 2 eine zylindrische Spitze mit einem deutlich geringeren Durchmesser als dem der Hülle (1) auf, deren oberes Ende halbkugelförmig ausgebildet ist. In diesem oberen Ende des Stopfens (3) ist der Schlitz des Stopfens (3) angeordnet. Bei dieser Vorrichtung wird die zylindrische Spitze in den Mund eingeführt und dann durch Druck auf die Hülle (1) die Flüssigkeit appliziert.

Bei dieser Vorrichtung wird die Schutzkappe (5) auf die zylindrische Spitze des Stopfens (3) aufgesteckt.

## Patentansprüche

1. Vorrichtung zum Applizieren von Flüssigkeiten, wobei die Vorrichtung eine zusammendrückbare hohlzylindrische Hülle (1) aufweist, welche an ihrem unteren Ende geschlossen und an ihrem oberen Ende offen ist, **dadurch gekennzeichnet, dass** in der hohlzylindrischen Hülle (1) ein saugfähiges Element (2) angeordnet ist und ein geschlitzter Stopfen (3) vorgesehen ist, der das offene Ende der hohlzylindrischen Hülle (1) verschließt, und dass in dem saugfähigen Element (2) ein perforiertes Steigrohr (4) angeordnet ist, das in den Schlitz des Stopfens (3) mündet.

2. Vorrichtung zum Applizieren von Flüssigkeiten, wobei die Vorrichtung eine feste hohlzylindrische Hülle (1) aufweist, welche an ihrem unteren Ende geschlossen und an ihrem oberen Ende offen ist, **dadurch gekennzeichnet, dass** in der hohlzylindrischen Hülle (1) ein saugfähiges Element (2) angeordnet ist und ein geschlitzter Stopfen (3) vorgesehen ist, der das offene Ende der hohlzylindrischen Hülle (1) verschließt, und dass in dem saugfähigen Element (2) ein perforiertes Steigrohr (4) angeordnet ist, das in den Schlitz des Stopfens (3) mündet.

3. Vorrichtung gemäß einem der Ansprüche 1 bis 2, **dadurch gekennzeichnet, dass** die hohlzylindrische Hülle (1) aus Kunststoff besteht.

4. Vorrichtung gemäß einem der Ansprüche 1 bis 3, **dadurch gekennzeichnet, dass** das saugfähige Element (2) aus Schaumstoff besteht.

5. Vorrichtung gemäß einem der Ansprüche 1 bis 4, **dadurch gekennzeichnet, dass** der Stopfen (3) halbkugelförmig ausgebildet ist.

6. Vorrichtung gemäß einem der Ansprüche 1 bis 5, **dadurch gekennzeichnet, dass** der Stopfen (3) aus einem gummielastischen Material besteht.

7. Vorrichtung gemäß einem der Ansprüche 1 bis 6, **dadurch gekennzeichnet, dass** das Steigrohr (4) über seine gesamte Länge perforiert ist.

8. Vorrichtung gemäß einem der Ansprüche 1 bis 7, **dadurch gekennzeichnet, dass** eine Schutzkappe (5) zum Verschließen der Vorrichtung vorgesehen ist.

9. Verwendung der Vorrichtung gemäß einem der Ansprüche 1 bis 8 zum Applizieren von Flüssigkeiten, **dadurch gekennzeichnet, dass** eine orale oder eine dermale Applikation erfolgt.

10. Verwendung der Vorrichtung gemäß Anspruch 1 zum Applizieren von Flüssigkeiten, **dadurch gekennzeichnet, dass** die Applikation durch das Zusammendrücken der hohlzylindrischen Hülle (1) erfolgt.

11. Verwendung der Vorrichtung gemäß Anspruch 2 zum Applizieren von Flüssigkeiten, **dadurch gekennzeichnet, dass** die Applikation durch Saugen an dem geschlitzten Stopfen (3), der das offene Ende der hohlzylindrischen Hülle (1) verschließt, erfolgt.

12. Vorrichtung gemäß einem der Ansprüche 1 bis 8 sowie Verwendung gemäß einem der Ansprüche 9 bis 11, **dadurch gekennzeichnet, dass** die Vorrichtung 1-30 ml einer mit der Vorrichtung zu applizierenden Flüssigkeit enthält und zur Mehrfachapplikation vorgesehen ist.

13. Vorrichtung gemäß einem der Ansprüche 1 bis 8 und 12 sowie Verwendung gemäß einem der Ansprüche 9 bis 11, **dadurch gekennzeichnet, dass** die Flüssigkeit eine homöopathische Flüssigkeit mit der folgenden Zusammensetzung ist:
| | |
|---|---|
| Passiflora incarnata D30 | 1,5 g |
| Rhus toxicodendron D60 | 0,8 g |
| Ignatia D30 | 1,0 g |
| Zincum metallicum D26 | 1,8 g |
| Cactus D30 | 1,1 g |
| Sulphur D6 | 0,9 g |
| Panax Ginseng D30 | 0,4 g |
| Pulsatilla D23 | 1,1 g |
| Iodum D30 | 0,9 g |
| Lobelia inflata D60 | 1,6 g |
| Stramonium D30 | 1,1 g |
| Nux Moschata D30 | 1,8 g |
| Agnus Cactus D4 | 1,1 g |
| Destilliertes Wasser | 80,0 g |
oder eine homöopathische Flüssigkeit mit der folgenden Zusammensetzung:
| | |
|---|---|
| Passiflora incarnata D30 | 1,5 g |
| Rhus toxicodendron D60 | 0,8 g |
| Robinia pseudoacacia D30 | 1,1 g |
| Ignatia D30 | 1,0 g |
| Zincum metallicum D26 | 1,8 g |
| Cactus D30 | 1,1 g |
| Sulphur D6 | 0,9 g |
| Panax Ginseng D30 | 0,4 g |
| Pulsatilla D23 | 1,1 g |
| Lobelia inflata D60 | 1,6 g |
| Chamomilla D30 | 0,9 g |
| Phosphorus D28 | 1,4 g |
| Stramonium D30 | 1,1 g |
| Nux vomica D30 | 0,7 g |
| Nux Moschata D30 | 1,8 g |
| Isotonische NaCl-Lösung | 80,0 g |
oder eine homöopathische Flüssigkeit mit der folgenden Zusammensetzung:
| | |
|---|---|
| Hypericum perforatum D1 | 0,01 g |
| Valeriana officinalis D1 | 0,01 g |
| Lupulinum (HAB 34) D1 | 0,01 g |
| [HAB, V. 4a, D1 mit Ethanol 86 % (m/m)] | |
| Vitex agnus-castus D1 | 0,01 g |
| Avena sativa D1 | 0,01 g |
| Coffea Arabica D1 | 0,01 g |
| Ethanol 62 % (m/m) | ad 10 g (entsprechend 12 ml) |

14. Vorrichtung gemäß Anspruch 13 zur Verwendung als Arzneimittel zur Raucherentwöhnungstherapie.

15. Verfahren zur Herstellung einer Vorrichtung gemäß einem der Ansprüche 1-8 und 12-14.

## Claims

1. Device for administering liquids, wherein the device has a compressible hollow-cylindrical sleeve (1) which is closed at its lower end and open at its upper end, **characterised in that** an absorbent element (2) is arranged in the hollow-cylindrical sleeve (1) and a slotted stopper (3) is provided which closes the open end of the hollow-cylindrical sleeve (1), and that a perforated riser tube (4) is arranged in the absorbent element (2) which opens into the slot of the stopper (3).

2. Device for administering liquids, wherein the device has a solid hollow-cylindrical sleeve (1) which is closed at its lower end and open at its upper end, **characterised in that** an absorbent element (2) is arranged in the hollow-cylindrical sleeve (1) and a slotted stopper (3) is provided which closes the open end of the hollow-cylindrical sleeve (1), and that a perforated riser tube (4) is arranged in the absorbent element (2) which opens into the slot of the stopper (3).

3. Device according to one of the claims 1 to 2, **characterised in that** the hollow-cylindrical sleeve (1) consists of plastic.

4. Device according to one of the claims 1 to 3, **characterised in that** the absorbent element (2) consists of foam material.

5. Device according to one of the claims 1 to 4, **characterised in that** the stopper (3) is hemispherical in form.

6. Device according to one of the claims 1 to 5, **characterised in that** the stopper (3) consists of an elastic material.

7. Device according to one of the claims 1 to 6, **characterised in that** the riser tube (4) is perforated over its entire length.

8. Device according to one of the claims 1 to 7, **characterised in that** a protective cover (5) is provided for sealing the device.

9. Use of the device according to one of the claims 1 to 8 for administering liquids, **characterised in that** an oral or a dermal administration takes place.

10. Use of the device according to claim 1 for administering liquids, **characterised in that** the administration is effected by compressing the hollow-cylindrical sleeve (1).

11. Use of the device according to claim 2 for administering liquids, **characterised in that** the administration is effected by sucking on the slotted stopper (3) which seals the open end of the hollow-cylindrical sleeve (1).

12. Device according to one of the claims 1 to 8 as well as use according to one of the claims 9 to 11, **characterised in that** the device contains 1-30 ml of a liquid to be administered with the device and is intended for multiple administrations.

13. Device according to one of the claims 1 to 8 and 12 as well as use according to one of the claims 9 to 11, **characterised in that** the liquid is a homoeopathic liquid with the following composition:
| | |
|---|---|
| Passiflora incarnata D30 | 1.5 g |
| Rhus toxicodendron D60 | 0.8 g |
| Ignatia D30 | 1.0 g |
| Zincum metallicum D26 | 1.8 g |
| Cactus D30 | 1.1 g |
| Sulphur D6 | 0.9 g |
| Panax Ginseng D30 | 0.4 g |
| Pulsatilla D23 | 1.1 g |
| Iodum D30 | 0.9 g |
| Lobelia inflata D60 | 1.6 g |
| Stramonium D30 | 1.1 g |
| Nux Moschata D30 | 1.8 g |
| Agnus Cactus D4 | 1.1 g |
| Distilled water | 80.0 g |
or a homeopathic liquid with the following composition:
| | |
|---|---|
| Passiflora incarnata D30 | 1.5 g |
| Rhus toxicodendron D60 | 0.8 g |
| Robinia pseudoacacia D30 | 1.1 g |
| Ignatia D30 | 1.0 g |
| Zincum metallicum D26 | 1.8 g |
| Cactus D30 | 1.1 g |
| Sulphur D6 | 0.9 g |
| Panax Ginseng D30 | 0.4 g |
| Pulsatilla D23 | 1.1 g |
| Lobelia inflata D60 | 1.6 g |
| Chamomilla D30 | 0.9 g |
| Phosphorus D28 | 1.4 g |
| Stramonium D30 | 1.1 g |
| Nux vomica D30 | 0.7 g |
| Nux Moschata D30 | 1.8 g |
| Isotonic Na Cl solution | 80.0 g |
or a homeopathic liquid with the following composition:
| | |
|---|---|
| Hypericum perforatum D1 | 0.01 g |
| Valeriana officinalis D1 | 0.01 g |
| Lupulinum (HAB 34) D1 | 0.01 g |
| [HAB, V. 4a, D1 with ethanol 86 % (m/m)] | |
| Vitex agnus castus D1 | 0.01 g |
| Avena sativa D1 | 0.01 g |
| Coffea Arabica D1 | 0.01 g |
| Ethanol 62 % (m/m) | ad 10 g(corresponding to 12 ml). |

14. Device according to claim 13 for use as a medicinal product for anti-smoking therapy.

15. Method for manufacturing a device according to one of the claims 1-8 and 12-14.

## Revendications

1. Dispositif d'application de liquides, dans lequel le dispositif présente une gaine cylindrique creuse comprimable (1) qui est fermée à son extrémité inférieure et ouverte à son extrémité supérieure, **caractérisé en ce qu'**un élément absorbant (2) est agencé dans la gaine cylindrique creuse (1) et il est prévu un bouchon fendu (3) qui ferme l'extrémité ouverte de la gaine cylindrique creuse (1), et **en ce qu'**un tuyau ascendant (4) perforé qui débouche dans la fente du bouchon (3) est agencé dans l'élément absorbant (2).

2. Dispositif d'application de liquides, dans lequel le dispositif présente une gaine cylindrique creuse rigide (1) qui est fermée à son extrémité inférieure et ouverte à son extrémité supérieure, **caractérisé en ce qu'**un élément absorbant (2) est agencé dans la gaine cylindrique creuse (1) et il est prévu un bouchon fendu (3) qui ferme l'extrémité ouverte de la gaine cylindrique creuse (1), et **en ce qu'**un tuyau ascendant (4) perforé qui débouche dans la fente du bouchon (3) est agencé dans l'élément absorbant (2).

3. Dispositif selon l'une quelconque des revendications 1 à 2, **caractérisé en ce que** la gaine cylindrique creuse (1) est en plastique.

4. Dispositif selon l'une quelconque des revendications 1 à 3, **caractérisé en ce que** l'élément absorbant (2) est en mousse.

5. Dispositif selon l'une quelconque des revendications 1 à 4, **caractérisé en ce que** le bouchon (3) est hémisphérique.

6. Dispositif selon l'une quelconque des revendications 1 à 5, **caractérisé en ce que** le bouchon (3) est en un matériau élastique comme du caoutchouc.

7. Dispositif selon l'une quelconque des revendications 1 à 6, **caractérisé en ce que** le tuyau ascendant (4) est perforé sur toute sa longueur.

8. Dispositif selon l'une quelconque des revendications 1 à 7, **caractérisé en ce qu'**un capuchon protecteur (5) est prévu pour fermer le dispositif.

9. Utilisation du dispositif selon l'une quelconque des revendications 1 à 8 pour appliquer des liquides, **caractérisée en ce qu'**il s'effectue une application orale ou une application cutanée.

10. Utilisation du dispositif selon la revendication 1 pour appliquer des liquides, **caractérisée en ce que** l'application s'effectue en comprimant la gaine cylindrique creuse (1).

11. Utilisation du dispositif selon la revendication 2 pour appliquer des liquides, **caractérisée en ce que** l'application s'effectue en aspirant au niveau du bouchon fendu (3) qui ferme l'extrémité ouverte de la gaine cylindrique creuse (1).

12. Dispositif selon l'une quelconque des revendications 1 à 8 ainsi qu'utilisation selon l'une quelconque des revendications 9 à 11, **caractérisés en ce que** le dispositif contient 1 à 30 ml d'un liquide à appliquer avec le dispositif et est prévu pour des applications multiples.

13. Dispositif selon l'une quelconque des revendications 1 à 8 et 12 ainsi qu'utilisation selon l'une quelconque des revendications 9 à 11, **caractérisés en ce que** le liquide est un liquide homéopathique avec la composition suivante :
| | |
|---|---|
| Passiflora incarnata D30 | 1,5 g |
| Rhus toxicodendron D60 | 0,8 g |
| Ignatia D30 | 1,0 g |
| Zincum metallicum D26 | 1,8 g |
| Cactus D30 | 1,1 g |
| Sulfur D6 | 0,9 g |
| Panax Ginseng D30 | 0,4 g |
| Pulsatilla D23 | 1,1 g |
| Iodum D30 | 0,9 g |
| Lobelia inflata D60 | 1,6 g |
| Stramonium D30 | 1,1 g |
| Nux Moschata D30 | 1,8 g |
| Agnus Cactus D4 | 1,1 g |
| Eau distillée | 80,0 g |
ou un liquide homéopathique avec la composition suivante :
| | |
|---|---|
| Passiflora incarnata D30 | 1,5 g |
| Rhus toxicodendron D60 | 0,8 g |
| Robinia pseudoacacia D30 | 1,1 g |
| Ignatia D30 | 1,0 g |
| Zincum metallicum D26 | 1,8 g |
| Cactus D30 | 1,1 g |
| Sulfur D6 | 0,9 g |
| Panax Ginseng D30 | 0,4 g |
| Pulsatilla D23 | 1,1 g |
| Lobelia inflata D60 | 1,6 g |
| Chamomilla D30 | 0,9 g |
| Phosphorus D28 | 1,4 g |
| Stramonium D30 | 1,1 g |
| Nux vomica D30 | 0,7 g |
| Nux Moschata D30 | 1,8 g |
| Solution NaCl isotonique | 80,0 g |
ou un liquide homéopathique avec la composition suivante :
| | |
|---|---|
| Hypericum perforatum D1 | 0,01 g |
| Valeriana officinalis D1 | 0,01 g |
| Lupulinum (HAB 34) D1 | 0,01 g |
| [HAB, V.4a, D1 avec 86 % d'éthanol (m/m)] | |
| Vitex agnus-castus D1 | 0,01 g |
| Avena sativa D1 | 0,01 g |
| Coffea Arabica D1 | 0,01 g |
| Éthanol 62 % (m/m) | ad 10 g (correspond à 12 ml) |

14. Dispositif selon la revendication 13 en vue de l'utilisation comme médicament pour la thérapie de désintoxication pour fumeurs.

15. Procédé de fabrication d'un dispositif selon l'une quelconque des revendications 1 à 8 et 12 à 14.
